Europäisches Patentamt

European Patent Office

Office européen des brevets

(19)

(11) **EP 1 057 017 B1**

(12) **EUROPÄISCHE PATENTSCHRIFT**

(45) Veröffentlichungstag und Bekanntmachung des Hinweises auf die Patenterteilung:
**06.10.2004  Patentblatt 2004/41**

(21) Anmeldenummer: **99913167.5**

(22) Anmeldetag: **17.02.1999**

(51) Int Cl.$^7$: **G01N 33/18**

(86) Internationale Anmeldenummer:
**PCT/EP1999/001024**

(87) Internationale Veröffentlichungsnummer:
**WO 1999/042824 (26.08.1999 Gazette 1999/34)**

(54) **VERFAHREN UND VORRICHTUNG ZUR BESTIMMUNG DES ORGANISCHEN KOHLENSTOFF(TOC-)GEHALTS IN FLÜSSIGKEITEN, INSBESONDERE REINSTWASSER**

METHOD AND DEVICE FOR DETERMINING THE TOTAL ORGANIC CARBON CONTENT IN LIQUIDS, ESPECIALLY ULTRA-PURE WATER

PROCEDE ET DISPOSITIF POUR DETERMINER LA TENEUR EN CARBONE ORGANIQUE TOTAL DANS DES LIQUIDES, NOTAMMENT DE L'EAU ULTRA-PURE

(84) Benannte Vertragsstaaten:
**AT BE CH DE DK ES FI FR GB GR IE IT LI LU NL PT SE**

(30) Priorität: **19.02.1998  DE 19806854**

(43) Veröffentlichungstag der Anmeldung:
**06.12.2000  Patentblatt 2000/49**

(73) Patentinhaber: **Maihak AG**
**22399 Hamburg (DE)**

(72) Erfinder:
• **VOSS, Werner**
**D-22393 Hamburg (DE)**

• **MESSERSCHMITT, Erich**
**D-67346 Speyer (DE)**
• **BENDLIN, Herbert**
**D-56235 Ransbach-Baumbach (DE)**

(74) Vertreter: **Jochem, Bernd , Dipl.-Wirtsch.-Ing et al**
**Patentanwälte Beyer & Jochem,**
**Klettenbergstrasse 13**
**60322 Frankfurt/Main (DE)**

(56) Entgegenhaltungen:
**EP-A- 0 150 923          EP-A- 0 498 888**
**WO-A-97/38304          FR-A- 2 124 744**
**US-A- 5 272 091**

**Beschreibung**

Die Erfindung betrifft ein Verfahren zur Bestimmung des Kohlenstoff (TOC-)Gehaltes in Flüssigkeiten, insbesondere Reinstwasser, wonach eine Probemenge der zu untersuchenden Flüssigkeit in eine Reaktionskammer eingeleitet und chargenweise durch UV-Bestrahlung zur Oxidation von Kohlenstoff zu im wesentlichen Kohlendioxid statisch behandelt wird, anschließend die Probemenge durch von außen in die Reaktionskammer eintretende Flüssigkeit in eine an die Reaktionskammer angeschlossene Meßzelle überführt wird, in der ihre Leitfähigkeit gemessen wird, und dann aus Leitfähigkeitsmeßwerten der Kohlenstoff (TOC-)Gehalt ermittelt wird.

Unter dem TOC-Gehalt einer Flüssigkeit wird der Gesamtgehalt an organischem Kohlenstoff (total organic carbon) verstanden. Die Erfassung dieses Kohleristoffgehaltes in insbesondere Reinstwasser ist von besonderer Bedeutung für moderne Hochtechnologien. An dieser Stelle sei hauptsächlich auf das für die Halbleiterproduktion zur Verfügung zu stellende Reinstwasser verwiesen. Aber auch in der pharmazeutischen Industrie kommt es auf eine zuverlässige Überwachung von Spuren organischer verschmutzungen an. Schon geringste Verschmutzungen durch Kohlenwasserstoffverbindungen, Aliphate und/oder aromatische Alkohole usw. müssen unbedingt vermieden werden. In diesem Zusammenhang sind in der Vergangenheit Meßmethoden und zugehörige -einrichtungen entwickelt worden, welche den Gehalt an organischem Kohlenstoff in Reinstwasser über einen weiten Bereich ermitteln können, welcher von einigen ppm (parts per million; $10^{-6}$) bis zu einigen ppb (parts per billion; $10^{-9}$) reicht.

Die US-A-3 958 941 beschreibt ein Verfahren, bei dem, mit Hilfe eines Zweikreissystems zunächst die organischen Bestandteile in der Reaktionskammer oxidiert werden, und zwar durch Bestrahlung mit UV-Licht. Das sich durch diese Maßnahme entwickelnde Kohlendioxid wird über einen getrennten (Luft-)Zuführungskanal in eine mit Reinstwasser gefüllte Meßzelle überführt. In dieser Meßzelle wird die Leitfähigkeit des Meßzelleninhaltes ermittelt, welche sich als Reaktion auf den Kohlendioxideinlaß erhöht.

Eine solche Vorgehensweise erfordert die Durchführung verschiedener vormessungen, um die Meßzelle bzw. die hier eingebrachten Leitfähigkeitsmeßeinrichtungen zu kalibrieren. Außerdem ist die Genauigkeit bei geringem Anteil an organischen Kohlenstoffverbindungen gering, weil Meßstörungen durch die UV-Bestrahlung nicht ausgeschlossen werden können. Im übrigen ist es zwingend erforderlich, das sich entwickelnde Kohlendioxid von der Reaktionskammer in die Meßzelle zu überführen und hier in dem Reinstwasser zu lösen. Derartiges ist nicht nur aufwendig, sondern führt auch zu unerwünschten Fehlerquellen beispielsweise dadurch, daß sich die Löslichkeit des Kohlendioxids in der Meßzelle temperaturabhängig ändert. Außerdem kann atmosphärisches Kohlendioxid, welches unbeabsichtigt in die Apparatur gelangt, die Meßgenauigkeit stören.

Diese vorerwähnten Nachteile hat man mit der europäischen Schrift 0 150 923 zu überwinden versucht. Hier wird in der Weise vorgegangen, daß der Kohlenstoffgehalt einer statischen Wasserprobe dergestalt ermittelt wird, daß diese Wasserprobe in eine Kammer mit Leitfähigkeitssensoren eingebracht und hierin statisch gehalten wird. Eine außen an dieser Kammer angeordnete UV-Lampe bestrahlt die wasserprobe so lange, bis die Oxidation der Kohlenstoffverbindungen abgeschlossen ist. Die sich einstellende Leitfähigkeitsdifferenz vor und nach der UV-Bescrahlung soll dann einen dem TOC-Gehalt äquivalenten Wert darstellen. Das insgesamt geschlossene System der EP 0 150 923 mag zwar zusammen mit der statischen Messung Einflüsse durch Verschleppung und Eintragung von Kohlendioxid aus der Atmosphäre verhindern. Allerdings wird dies mit dem Nachteil erkauft, daß die außerhalb der Kammer bzw. Meßzelle angeordnete UV-Lampe durch den relativ kleinen Abstrahlwinkel nur sehr wenig wirksame Strahlung in die Meßzelle bzw. Oxidationskammer einkoppeln kann. Außerdem ist für den optisch durchlässigen Verschluß der Meßzelle ein Quarzfenster vorgesehen, welches zu Strahlungsverlusten durch Absorption und Reflektion korrespondiert. Diese addieren sich zu den ohnehin nicht zu vermeidenden Verlusten, die sich auf die Quarzglasumhüllung der UV-Lampe zurückführen lassen.

Außerdem ist durch die in der Oxidationskammer bzw. Meßzelle angebrachten Meßelektroden eine große Schichtdicke des zu bestrahlenden Wassers unerläßlich, welche über die wirksame Eindringtiefe der UV-Strahlung hinausgeht. Das hat zur Folge, daß die organischen Inhaltsstoffe des nicht bewegten Wassers in der Kammer nur sehr unvollständig und langsam oxidiert werden. Obwohl mit der bekannten Einrichtung TOC-Konzentrationen im ppb-Bereich ermittelt werden können, führt dies - gerade im untersten Meßbereich - zu hier unerwünschten hohen Meßfehlern.

Tatsächlich sind Oxidationszeiten bis zu ca. 10 Minuten erforderlich, die letztlich zu Leitfähigkeitswerten führen, die sich asymptotisch einem Grenzwert annähern, der mathematisch ermittelt werden muß. Jedenfalls muß die Leitfähigkeitsmessung ausschließlich bei eingeschalteter UV-Lampe erfolgen, welches - wie bei der US-PS 3 958 941 - erhebliche Störungen in die notwendigerweise erforderliche Elektronik einträgt. Denn UV-Lampen werden üblicherweise mit Hochspannung im Bereich von mehreren 100 Volt betrieben.

Ein weiterer Nachteil der EP 0 150 923 ist darin zu sehen, daß die Leitfähigkeitsmessung durch den relativ großen Wasserinhalt der Meßzelle bzw. Oxidationskammer langsam reagiert, folglich eine große Zeitkonstante aufweist. Außerdem bedingt der bekannte konstruktive Aufbau, daß sich hinter den Meßelektroden Schattenbereiche bilden können, die nicht von der UV-Strahlung erreicht werden. Hinzu kommt, daß die UV-Lampe mit zunehmender Brenndauer eine erhöhte Temperatur aufweist, so daß nicht nur ein Kühlkörper erforderlich ist, sondern die Überhitzung auch zu unerwünschten Meßfehlern führen kann. Denn die ermittelte Leitfähigkeit ist nicht nur eine Funktion der Kohlendioxidkonzentration im Wasser sondern auch der Wassertemperatur. Folglich kommt es darauf an, die Flüssigkeit bzw. das Wasser in der Meßzelle auf möglichst konstanter Temperatur zu halten, was bei der bekannten Lehre aufgrund der geschilderten konstruktiven Besonderheiten nicht gegeben ist und somit eine schnelle Temperaturmessung und ggf. -kompensation erforderlich ist. Hinzu kommt ein weiterer (indirekter) Effekt, welcher sich daraus erklärt, daß die Strahlintensität einer regelmäßig eingesetzten Quecksilberdampflampe zur Erzeugung der UV-Strahlung mit steigender Temperatur abnimmt. Jedenfalls treten durch die langen Oxidationszeiten erhebliche Probleme auf, die zur Erhöhung der Meßgenauigkeit unbedingt vermieden werden müssen.

Darüber hinaus ist es durch die deutsche Offenlegungsschrift 32 23 167 bekannt, Wasser kontinuierlich hinsichtlich zersetzungsfähiger organischer und/oder anorganischer Kohlenstoff-Verbindungen zu untersuchen. Zu diesem Zweck wird das zu untersuchende Wasser kontinuierlich durch eine Bestrahlungszelle durchgeleitet, wobei hier der pH-Wert im Bereich von 7,0 bis 7,3 eingestellt wird. Anschließend erfolgt eine Bestrahlung des zu untersuchenden Wassers mit UV-Licht und ein kontinuierliches Überleiten des Gasgemisches, welches die flüchtigen Zersetzungsprodukte enthält, aus der Bestrahlungszelle in eine Meßzelle. Die vorgenannten flüchtigen Zersetzungsprodukte werden durch ein Kreisgas aus dem zu untersuchenden Wasser kontinuierlich teilweise ausgetrieben. Danach wird die IR(Infrarot)-Absorption des Kreisgases gemessen und/oder das Kreisgas wird in eine kontinuierlich durchströmte Leitfähigkeitszelle geleitet und dort teilweise gelöst, wodurch sich die Leitfähigkeit des Wassers ändert. Zum Nachweis der organischen Kohlenstoffverbindungen brauchen die anorganischen Kohlenstoffverbindungen, die üblicherweise durch Säure zersetzt werden, vorher nicht entfernt zu werden. Mithin wird für den Fall der Bestimmung des organischen Kohlenstoffgehaltes praktisch so vorgegangen wie bei der eingangs beschriebenen US-PS 3 958 941, so daß sich die gleichen Nachteile wie bei dieser vorbeschriebenen Lehre ergeben.

Schließlich ist aus der US-Patentschrift 5,272,091 eine Vorrichtung zur Erzeugung von Reinstwasser bekannt, die im Hauptstrom eine Oxidationskammer aufweist, in der das gesamte zu reinigende Wasser während des Hindurchströmens mit UV-Strahlen behandelt wird. Gleichzeitig wird vor der Oxidationskammer in einer Meßzelle ein Grundwert der Leitfähigkeit und hinter der Oxidationkammer in einer weiteren Meßzelle ein infolge der Bestrahlung erhöhter Leitfähigkeitswert gemessen. Die Differenz zwischen diesem und dem Grundwert ist ein Maß für den durch die UV-Bestrahlung beseitigten Gehalt an organischem Kohlenstoff. Da aber je nach der Art der Kohlenstoffverbindungen und anderer Einflußfaktoren die Leitfähigkeitswerte unterschiedlich ausfallen können, wird von Zeit zu Zeit die Erzeugung von Reinstwasser unterbrochen, um nacheinander mehrere Füllmengen der Oxidationskammer chargenweise jeweils unterschiedlich lange der UV-Bestrahlung auszusetzen. Nach jeder Bestrahlung wird der Hauptstrom des Wassers vorübergehend wieder durch die Oxidationskammer gepumpt und in der stromabwärts anschließenden Meßzelle ein der Bestrahlungsdauer jeweils zugeordneter Leitwert gemessen. Aus der Reihe der Messungen läßt sich dann rechnerisch ein Referenzwert für den Leitfähigkeitsanstieg bei Zersetzung des gesamten organischen Kohlenstoffgehalts gewinnen und dieser mit dem während der anschließenden Behandlung des kontinuierlich strömenden Wassers durch Messungen vor und hinter der Oxidationskammer ermittelten Leitfähigkeitsanstieg vergleichen.

Das zuletzt genannte bekannte Verfahren hat im Vergleich zu Meßverfahren mit. Reaktionskammern und Meßzellen, die nicht im Hauptstrom einer Anlage zur Behandlung von Wasser angeordnet sind und somit nur Probemengen aufzunehmen brauchen, den Nachteil größerer Ungenauigkeit, weil im Hauptstrom alle Strömungsquerschnitte groß sein müssen und es sehr lange dauern würde, wenn man den gesamten organischen Kohlenstoff in dem großen Wasservolumen der Oxidationskammer durch UV-Strahlen zersetzen wollte. Grundsätzlich wirkt sich hier die bereits erwähnte Problematik besonders stark aus, daß die UV-Strahlen nur bis in eine geringe Wassertiefe wirksam sind. Daher wird bei der bekannten Vorrichtung jeweils nur verhältnismäßig kurzzeitig bestrahlt, also grundsätzlich auf vollständige Oxidation des organischen Kohlenstoffs verzichtet und stattdessen der Leitfähigkeits-Referenzwert durch Extrapolation ermittelt, womit eine weitere Ungenauigkeit verbunden ist. Schließlich ist auch die Genauigkeit des Meßvorgangs in der Meßzelle beeinträchtigt, weil ihre Querschnitte ebenfalls der Strömungsrate des Hauptstroms angepaßt sind. Darüber hinaus wird bei jeder chargenweisen Messung nur ein einziger Leitfähigkeits-Meßwert bestimmt und nicht beim Ausschieben des in der Oxidationskammer bestrahlten Wasservolumens ein auszuwertendes Leitfähigkeitsprofil der ausströmenden Wassersäule ermittelt. Letzteres würde daran scheitern, daß sich das unterschiedlich intensiv bestrahlte Wasser auf dem Weg in die Meßzelle innerhalb der großen Strömungsquerschnitte vermischt, so daß ein die vollständige Zerstörung des organischen Kohlenstoffgehalts repräsentierender Peak sich nicht darstellen würde.

Der Erfindung liegt die Aufgabe zugrunde, ein Verfahren der zuletzt beschriebenen Art so weiterzubilden, daß bei einfachem Aufbau und hoher Oxidationswirkung der Kohlenstoffgehalt der zu untersuchenden Flüssigkeit schnell und zuverlässig sowie mit geringen Meßfehlern - auch im ppb-Bereich - ermittelt werden kann. Außerdem soll eine besonders geeignete Vorrichtung geschaffen werden.

Zur Lösung dieser Aufgabe schlägt die Erfindung bei einem gattungsgemäßen Verfahren vor, daß die Probemenge im Anschluß an die mittels einer UV-Tauchlampe durchgeführte Kohlenstoffoxidation durch von außen in die Reaktionskammer eintretende Flüssigkeit in eine an die Reaktionskammer angeschlossene - von der Reaktionskammer getrennte - Leitfähigkeitsmeßzelle überführt und hier die zum Kohlenstoffgehalt proportionale Kohlendioxidkonzentration der Flüssigkeit im Zuge des Durchströmens der Meßzelle dynamisch erfaßt wird. Dabei wird üblicherweise so vorgegangen, daß vor und/oder nach Ermittlung der Kohlendioxidkonzent-ration die Reaktionskammer und die Meßzelle - selbstverständlich ohne Beaufschlagung mit externer Energie - mittels durchströmender Flüssigkeit gereinigt werden. Bei dieser durchströmenden Flüssigkeit handelt es sich üblicherweise um die zu untersuchende Flüssigkeit, die im allgemeinen auch als von außen in die Reaktionskammer nach der Kohlenstoffoxidation eintretende Flüssigkeit zum Einsatz kommt. Jedenfalls lassen sich vor Beginn der Oxidation des Kohlenstoffs und/oder nach Ermittlung der Kohlendioxidkonzentration in der zu untersuchenden Flüssigkeit definierte Werte für die Kohlendioxidkonzentration angeben. Diese sollte möglichst gering sein.

Bei der vorliegenden Erfindung wird die Kohlendioxidkonzentration in der Flüssigkeit anhand von Leitfähigkeitsmessungen bestimmt. Hierbei geht die Erfindung von der Erkenntnis aus, daß gelöstes Kohlendioxid in einer Flüssigkeit, üblicherweise Wasser, den Widerstand aufgrund der sich bildenden Wasserstoff- und Carbonationen verringert, so daß sich die Leitfähigkeit erhöht.

Im Ergebnis können jedenfalls Leitfähigkeitsmeßwerte angegeben werden, welche im Rahmen der Erfindung als zur unbehandelten Flüssigkeit korrespondierender Grundwert der Kohlendioxidkonzentration bezeichnet werden. Dieser Grundwert wird ebenso wie ein sich im Zuge des Durchflusses der mit Kohlendioxid angereicherten Flüssigkeit durch die Meßzelle einstellender Maximalwert der Kohlendioxidkonzentration gemessen. Anschließend wird regelmäßig die zum Kohlenstoffgehalt in der Flüssigkeit proportionale Differenz aus Maximalwert und Grundwert gebildet (unter Berücksichtigung von Nichtlinearitäten bei der vorgenannten Abhängigkeit).

Zusammenfassend macht sich die Erfindung zunutze, daß die unbehandelte Flüssigkeit zu einem Grundwert der Leitfähigkeit korrespondiert, welcher sich dem Maximalwert der Leitfähigkeit bzw. der Kohlendioxidkonzentration annähert, sobald das zuvor in der Reaktionskammer behandelte Flüssigkeitvolumen im Zuge des Durchströmens die Meßzelle passiert. Man kann sich vorstellen, daß das statisch mit externer Energie zur Oxidation des Kohlenstoffs beaufschlagte Flüssigkeitsvolumen der Probemenge praktisch als Flüssigkeitssäule durch die von außen in die Reaktionskammer eintretende Flüssigkeit in die an die Reaktionskammer angeschlossene Meßzelle "weitergeschoben" wird. Diese behandelte Flüssigkeitssäule durchscrömt nun die Meßzelle, wobei die zum Kohlenstoffgehalt proportionale Kohlendioxidkonzentration - also vorliegend die Leitfähigkeit - dynamisch erfaßt wird. Der zugehörige zeitliche Verlauf des Meßsignals der Leitfähigkeit stellt also einen ansteigenden und anschließend abfallenden "peak" dar, dessen Maximum unschwer ermittelt werden kann. Dies läßt sich üblicherweise dergestalt realisieren, daß in einer an einen Leitfähigkeitssensor angeschlossenen Steuer-/Auswerteeinrichtung zeitliche Ableitungen des Meßsignals ermittelt werden, also die Steigung durch Differenzieren nach der Zeit bestimmt wird. Sobald diese Steigung den Wert Null erreicht, liegt der Maximalwert der Leitfähigkeit vor. Dieser Maximalwert der Leitfähigkeit kann in Beziehung zum Grundwert der Leitfähigkeit bzw. Kohlendioxidkonzentration durch Differenzbildung gesetzt werden. Diese Differenz ist ein Maß für die im Zuge der Oxidation erzeugte Kohlendioxidkonzentracion in der zu untersuchenden Flüssigkeit. Aus dieser Kohlendioxidkonzentration läßt sich unschwer der Kohlenstoffgehalt der zu untersuchenden Flüssigkeit ermitteln. Hierzu sei nur beispielhaft auf die EP 0 150 923 und den dort zitierten Artikel "A New Anproach to the Measurement of Organic Carbon" von Poirier et al. (American Laboratory, December 1978) verwiesen.

Schließlich wird die externe Energie zur Oxidation des Kohlenstoffs in der zu untersuchenden Flüssigkeit vorzugsweise mittels einer in die zu untersuchende Flüssigkeit in der Reaktionskammer eintauchenden UV-Lampe aufgebracht. Bei dieser UV-Lampe kann es sich um eine Quecksilberdampflampe handeln. Üblicherweise emittieren derartige Strahlungsquellen im Wellenlängenbereich von 10 bis 380 nm, vorzugsweise zwischen 170 und 260 nm.

Durch diese erfindungsgemäßen Maßnahmen wird zunächst einmal 1 bei einfachem Aufbau eine hohe Oxiationswirkung erreiche. Dies läßt sich schlicht und einfach darauf zurückführen, daß nach bevorzugter Ausführungsform eine UV-Tauchlampe in die Reaktionskammer eingetaucht wird und in direktem Kontakt mit der zu untersuchenden Flüssigkeit in der Reaktionskammer steht. In diesem Zusammenhang kann mit geringen Probemengen und folglich kleinen Querschnittslängen der Reaktionskammer gearbeitet werden, so daß die Querschnittslänge auf jeden Fall an die Eindringtiefe der UV-Lampe anpaßbar ist. Außerdem entfallen durch die direkte Einkopplung der UV-Lampe in die Reaktionskammer abdichtende Quarzfenster, so daß Absorptions- und Reflektionsverluste minimiert sind. Diese Vorgehensweise hat weiter zum Vorteil, daß auf eine zusätzliche Kühlung der UV-Lampe verzichtet werden kann, da aufgrund der geringen Eindringtiefe und der hohen Strahlintensität kurze Oxidationszeiten erreichbar sind. Außerdem wird für eine gleichmäßige Temperierung der UV-Lampe durch die vor- und nachgeschalteten Reinigungsschritte mit durchströmender Flüssigkeit gesorgt.

Ferner ist eine schnelle und zuverlässige Bestimmung der Kohlendioxidkonzentration anhand von Leitfähigkeitsmessungen möglich, da es letztlich nur auf die Ermittlung des Grundwertes und des Maximalwertes der Kohlendioxidkonzentration bzw. Leitfähigkeit ankommt. Eine derartige Messung kann problemlos, schnell und ohne gravierende Meßfehler durchgeführt werden, weil hierdurch gleichsam Untergrundeffekte ausgeschlossen sind. Die Temperaturkonstanz der UV-Tauchlampe führt darüber hinaus zu einer nochmaligen Verringerung der Meßfehler, da mit einem Abfall der Strahlintensität durch Temperatureffekte ebenso wenig wie mit einer Erwärmung der in der Reaktionskammer behandelten Flüssigkeit zu rechnen ist. Folglich können Temperatureffekte bei der Leitfähigkeitsmessung ausgeschlossen werden. Schließlich ist zu berücksichtigen, daß durch die Vornahme der Messung bei ausgeschalteter UV-Lampe Störungen des oder der Leitfähigkeitssensoren sowie der Steuer-/Auswerteeinrichtung zuverlässig verhindert werden. Hinzu kommt, daß die Meßzelle hinsichtlich ihrer meßtechnischen Eigenschaften optimiert werden kann und auf gute UV-Strahlungsdurchlässigkeit keine Rücksicht zu nehmen ist. Durch den ständigen Kontakt der UV-Lampe mit dem zu messenden Wasser bzw. der Flüssigkeit ist keine zusätzliche Wärmeableitung über Kühlrippen notwendig, so daß nicht nur die Temperaturkonstanz verbessert wird, sondern zugleich Kostenvorteile geltend gemacht werden können. Immer arbeitet die UV-Lampe im optimalen Arbeitstemperaturbereich, d. h. im Temperaturbereich mit maximaler Strahlungsintensität. Endlich darf nicht unerwähnt bleiben, daß ein Wechsel der UV-Lampe problemlos möglich ist, weil es sich - wie gesagt - um eine UV-Tauchlampe handelt, die in einer von der Meßzelle unabhängigen und generell einfach gestalteten Reaktionskammer angeordnet ist. Hierin sind die wesentlichen Vorteile der Erfindung zu sehen.

Gegenstand der Erfindung ist auch eine Vorrichtung zur Bestimmung des organischen Kohlenstoff (TOC-)Gehaltes in Flüssigkeiten nach Patentanspruch 5. Vorteilhafte Ausgestaltungen dieser Vorrichtung sind in den Patentansprüchen 6 bis 14 beschrieben.

Im folgenden wird die Erfindung anhand einer lediglich ein Ausführungsbeispiel darstellenden Zeichnung näher erläutert; es zeigen:

Fig. 1    eine erfindungsgemäße Vorrichtung in schematischer Darstellung,

Fig. 2    zeigt das in der Meßzelle gemessene zeitabhängige Leitfähigkeitsmeßsignal und

Fig. 3    stellt die ermittelten Leitfähigkeitsdifferenzen in Abhängigkeit von der- Oxidationszeit bei unterschiedlichen TOC-Konzentrationen dar.

In den Figuren ist eine Vorrichtung zur Bestimmung des organischen Kohlenstoff(TOC-)Gehaltes in Flüssigkeiten, nach dem Ausführungsbeispiel in Reinstwasser, gezeigt. Diese Vorrichtung weist in ihrem grundsätzlichen Aufbau eine Reaktionskammer 1 mit Einlaß 2 zur Aufnahme einer Probemenge der zu untersuchenden Flüssigkeit und statischen Oxidation des hierin gelösten Kohlenstoffes auf. Zusätzlich ist eine an die Reaktionskammer 1 angeschlossene - von der Reaktionskammer 1 getrennte - Meßzelle 3 vorgesehen. Ausgangsseitig der Meßzelle 3 befindet sich eine Absperreinrichtung 4, im Ausführungsbeispiel ein elektromagnetisch steuerbares Ventil 4. Dieses Ventil 4 bzw. die Absperreinrichtung 4 ist nach Einleitung der Probemenge der zu untersuchenden Flüssigkeit in die Reaktionskammer 1 schließbar und nach statischer Oxidation des in der Flüssigkeit gelösten Kohlenstoffs zu Kohlendioxid in der Reaktionskammer 1 öffenbar. Die zum Kohlenstoffgehalt proportionale Kohlendioxidkonzentration der Flüssigkeit läßt sich im Zuge des Durchströmens der Meßzelle 3 dynamisch erfassen. Hierzu ist nach dem Ausführungsbeispiel ein Leitfähigkeitssensor 5 in der Meßzelle 3 vorgesehen. Dieser Leitfähigkeitssensor 5 ist an eine Steuer-/Auswerteeinrichtung 6 angeschlossen.

Im einzelnen wird die Probemenge nach beendeter Oxidation in der Reaktionskammer 1 und nach Öffnen des Ventils 4 durch von außen in die Reaktionskammer 1 eintretende Flüssigkeit in die angeschlossene Meßzelle 3 überführt.

Zur Oxidation des Kohlenstoffs mittels externer Energie steht eine UV-Lampe 7 zur

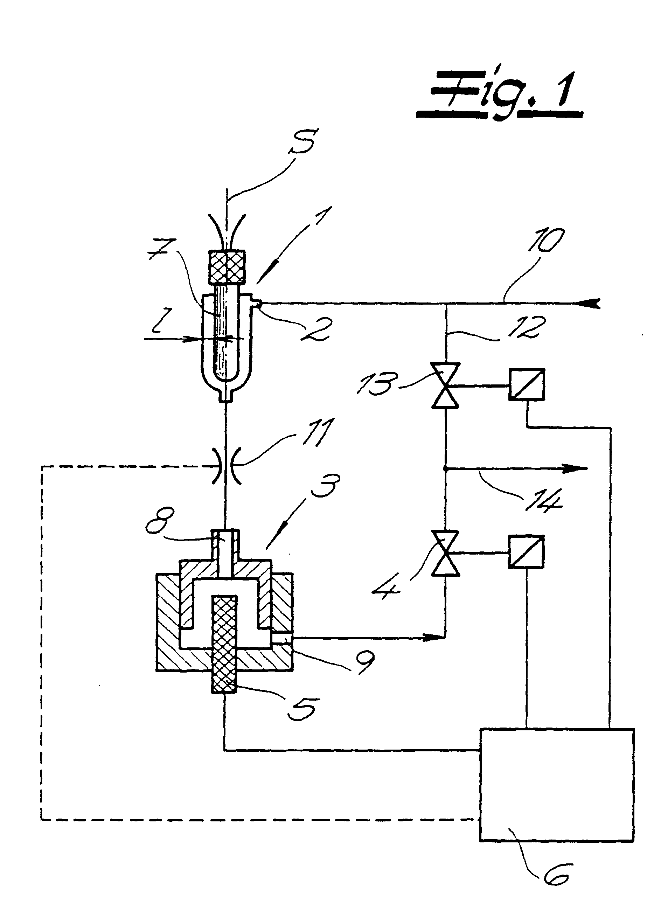

Sie ist als UV-Tauchlampe 7 ausgeführt und bildet mit der Reaktionskammer 1 eine kompakte, zusammengehörige Baueinheit. Die UV-Tauchlampe 7 ist mittig auf einer Symmetrieachse S angeordnet, wobei die Reaktionskammer 1 und die Tauchlampe 7 in bezug auf die Symmetrieachse S rotationssymmetrisch ausgebildet sind. Auch die Meßzelle 3 ist ausweislich der Fig. 1 (rotations-)symmetrisch ausgebildet und weist einen Zufluß 8 sowie einen Abfluß 9 mit jeweils gleich großem Querschnitt auf. Folglich strömt die oxidierte und zu untersuchende Flüssigkeit mit gleichmäßiger Geschwindigkeit durch die Meßzelle 3 mit Zufluß 8 und Abfluß 9.

Die rotationssymmetrisch ausgebildete Reaktionskammer 1 weist die Form eines rotierenden U-Rohrs mit vorzugsweise 1 bis 5 mm Querschnittslänge 1 auf. Diese Querschnittslänge 1 ist an die Eindringtiefe der UV-Tauchlampe 7 in das zu oxidierende bzw. zu untersuchende Wasser bzw. die Flüssigkeit angepaßt. Diese, d. h. die Flüssigkeit bzw. Probemenge wird im übrigen durch eine Zuleitung 10 über den Einlaß 2 in die Reaktionskammer 1 eingeleitet.

Zur Steuerung der Durchflußrate durch die Meßzelle 3 ist zwischen Reaktionskammer 1 und Meßzelle 3 ein vorzugsweise einstellbares Reduzierventil 11 zwischengeschaltet. Schließlich befindet sich parallel zur Reaktionskammer 1 und Meßzelle 3 eine Bypassleitung 12 mit steuerbarem Ventil 13. Bei diesem Ventil 13 handelt es sich nach dem Ausführungsbeispiel ebenfalls - wie bei der Absperreinrichtung 4 - um ein elektromagnetisch steuerbares Ventil 13. Sämtliche Ventile 4, 13 sind zusammen mit dem Leitfähigkeitssensor 5 an die Steuer-/Auswerteeinrichtung 6 angeschlossen. Nach einer alternativen Ausführungsform kann auch das einstellbare Reduzierventil 11 mit der Steuer-/Auswerteeinrichtung 6 verbunden werden, so daß sich über diese Einrichtung die Durchflußrate durch die Meßzelle 3 durch Änderung des Querschnittes des Reduzierventils 11 einstellen läßt.

Die Arbeitsweise der vorstehend beschriebenen Vorrichtung wird im folgenden erläutert. Die zu untersuchende Flüssigkeit bzw. das Reinstwasser nach dem Ausführungsbeispiel wird hinsichtlich seines Kohlenstoff(TOC-)Gehaltes dergestalt untersucht, daß eine Probemenge dieser Flüssigkeit in die Reaktionskammer 1 eingeleitet und getrennt von der Flüssigkeit (d. h. bei geschlossenem Ventil 4) mit UV-Strahlungsenergie durch die UV-Tauchlampe 7 zur Oxidation des Kohlenstoffs zu im wesentlichen Kohlendioxid statisch beaufschlagt wird. Bevor eine entsprechende Probemenge in die Reaktionskammer 1 eintritt, werden zunächst Reaktionskammer 1 und Meßzelle 3 mittels durchfließender Flüssigkeit gereinigt. Ein solcher Reinigungsschritt kann auch nach Ermittlung der Kohlendioxidkonzentration erfolgen. Jedenfalls ist während dieser Zeit die UV-Tauchlampe 7 abgeschaltet.

Im einzelnen tritt das Wasser über die Zuleitung 10 und den Einlaß 2 in die Reaktionskammer 1 ein und gelangt von dort über das Reduzierventil 11 in die Meßzelle 3, um schließlich über den dortigen Abfluß 9 und das Ventil 4 in Pfeilrichtung wieder auszutreten. Bei diesem Reinigungsschritt ist das Ventil 13 geschlossen. Der erforderliche Druck zur Sicherstellung des Durchflusses wird durch den üblicherweise in Reinstwasseraufbereitungssystemen herschenden Überdruck zur Verfügung gestellt.

Nach Schließen der Ventile 4 und 13 erfolgt durch Einschalten der UV-Tauchlampe 7 die Oxidation der organischen Inhaltsstoffe in der in der Reaktionskammer 1 eingeschlossenen Probemenge, und zwar unter statischen Bedingungen. Nach Abschluß der Oxidation und Ausschalten der UV-Tauchlampe 7 werden beide Magnetventile 4 und 13 geöffnet. Dies führt dazu, daß ein Teil des über die Zuleitung 10 einströmenden Wassers über den Bypass 12 und das Ventil 13 zur Ableitung 14 gelangt. Ein kleinerer Teil des eintretenden Wassers strömt über das Reduzierventil 11 durch die Meßzelle 3. Im Detail sorgt dieses von außen in die Reaktionskammer 1 eintretende Wasser bzw. die Flüssigkeit dafür, daß die (oxidierte) Probemenge von der Reaktionskammer 1 in die angeschlossene Meßzelle 3 über das Reduzierventil 11 überführt wird. In dieser Meßzelle 3 erfolgt anschließend die Erfassung der zum Kohlenstoffgehalt proportionalen Kohlendioxidkonzentration im Zuge des Durchströmens der Meßzelle 3, das heißt dynamisch. Im Anschluß hieran gelangt die Probemenge über den Abfluß 9 und das Ventil 4 in die Ableitung 14.

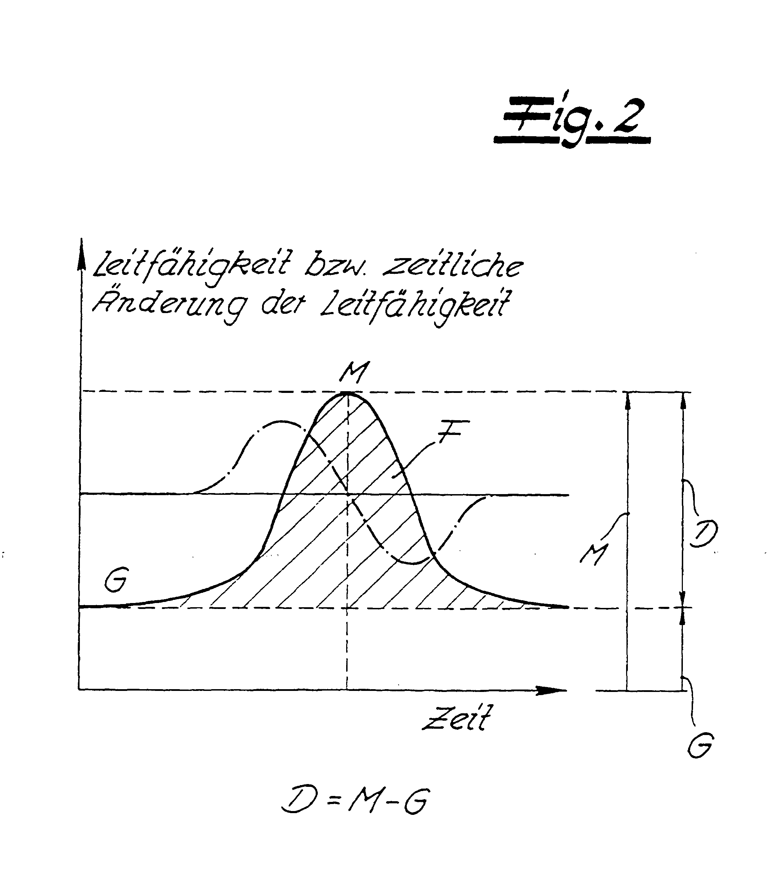

Der Leitfähigkeitssensor 5 ermittelt beim Durchströmen der Probemenge ein Leitfähigkeitssignal, wie es schematisch in der Fig. 2 dargestellt ist. Das heißt, ausgehend von einem zu der unbehandelten Flüssigkeit bzw. des Wassers korrespondierenden Grundwert G, stellt sich im Zuge des Durchflusses der mit Kohlendioxid angereicherten Flüssigkeit bzw. der Probemenge ein Maximalwert M der Kohlendioxidkonzentration bzw. der Leitfähigkeit ein (vgl. die durchgezogene Kurve in Fig. 2). Mittels der Steuer-/Auswerteeinrichtung 6 läßt sich dieser Maximalwert M unschwer durch zeitliches Differenzieren des Leitfähigkeitssignals ermitteln. Dieses differenzierte zeitabhängige Leitfähigkeitssignal ist in der Fig. 2 schematisch strichpunktiert angedeutet. Der Maximalwert M korrespondiert nun zum Nulldurchgang dieses "Ableitungssignals". Aus dem Maximalwert M und dem Grundwert G läßt sich unter Berücksichtigung von Nichtlinearitäten die zum Kohlenstoffgehalt in der Flüssigkeit proportionale Differenz

$$D = M - G \qquad\qquad (1.1)$$

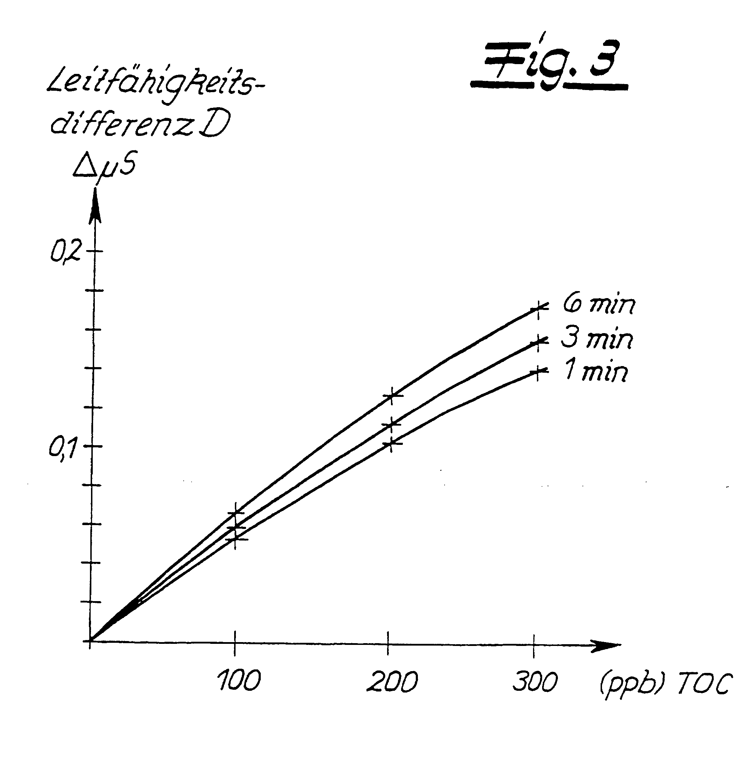

bilden. Diese Differenz D eignet als Maß für den Kohlenstoff(TOC-)Gehalt, wie bereits ausgeführt wurde. Tatsächlich besteht zwischen D und TOC eine größtenteils lineare Abhängigkeit mit bekannten (nichtlinearen) Abweichungen (vgl. Fig. 3). - Genauere Berechnungsmethoden gehen von der in Fig. 2 schraffiert dargestellten Fläche F anstelle des berechneten Wertes für D nach Gleichung 1.1 zur Bestimmung des TOC-Wertes aus. Diese Fläche F stellt das Integral über das um den Grundwert G verringerte Leitfähigkeitssignal dar. In diesem Fall gilt gleichsam F = D. Entsprechende Leitfähigkeitsdifferenzen D sind über die hierzu korrespondierenden Kohlenstoffkonzentrationen TOC in der Fig. 3 aufgetragen.

Die nur geringe Querschnittslänge 1 in der Reaktionskammer 1 und der direkte Kontakt der Flüssigkeit bzw. des Wassers zur UV-Tauchlampe 7 an dieser Stelle führen zu einer kurzen Oxidationszeit, da Absorption- und Reflektionsverluste minimiert sind. Diese Oxidation ist nach ca. 1 Minute für alle (relevanten) organischen Komponenten vollständig abgeschlossen. Eine solche Wertung ergibt sich unmittelbar aus der Fig. 3, welche die Leitfähigkeitsdifferenzen D gegenüber der Konzentration TOC (in ppb) darstellt, und zwar in Abhängigkeit von drei verschiedenen Oxidationszeiten (1 Minute, 3 Minuten und 6 Minuten). Die sich ändernden Leitfähigkeitsdifferenzen D bei längeren Oxidationszeiten (3 Minuten und 6 Minuten gegenüber 1 Minute) werden größtenteils durch die sich einstellende höhere Temperatur des Wassers bzw. der Flüssigkeit verursacht.

Mit Hilfe der Steuer-/Auswerteeinrichtung 6 lassen sich schließlich nicht nur der Maximalwert M entsprechend Fig. 2 sowie die zugehörige Differenz D=M-G ermitteln. Vielmehr erlaubt diese Einrichtung auch die Steuerung der Durchflußrate durch die Meßzelle 3 im Sinne einer Querschnittsveränderung des Reduzierventiles 11. Das heißt, letztlich läßt sich das Verhältnis des Flusses durch die Reaktionskammer 1 und die Meßzelle 3 im Vergleich zu dem Fluß durch den Bypass 12 einstellen. Mit Hilfe dieser Vorgehensweise kann gleichsam die Zeitachse in Fig. 2 variiert werden, da sich die Geschwindigkeit der zur Probemenge korrespondierenden "Probesäule" hiermit verändern läßt. Folglich kann der in Fig. 2 gezeigte "peak" zeitlich mehr oder minder "auseinander gezogen" werden, je nachdem wie hoch die Durchflußgeschwindigkeit und damit die Durchflußrate ist. Jedenfalls erlaubt diese Vorgehensweise eine eindeutige Bestimmung des Maximums M - auch bei geringen Kohlenstoff(TOC-)Gehalten.

Der in Fig. 2 dargestellte Meßablauf sieht zunächst ein ca. 3-minütiges Spülen, anschließend ein ca. 1½-minütiges Oxidieren und dann das gezeigte Messen vor. Danach erfolgt ein wiederum 3-minütiges Spülen.

**Patentansprüche**

1. Verfahren zur Bestimmung des Kohlenstoff (TOC-) Gehaltes in Flüssigkeiten, insbesondere Reinstwasser, wonach eine Probemenge der zu untersuchenden Flüssigkeit in eine Reaktionskammer (1) eingeleitet und chargenweise durch UV-Bestrahlung zur Oxidation von Kohlenstoff zu im wesentlichen Kohlendioxid statisch behandelt wird, anschließend die Probemenge durch von außen in die Reaktionskammer (1) eintretende Flüssigkeit in eine an die Reaktionskammer (1) angeschlossene Meßzelle (3) überführt wird, in der ihre Leitfähigkeit gemessen wird, und dann aus Leitfähigkeitsmeßwerten der Kohlenstoff (TOC-) Gehalt ermittelt wird, **dadurch gekennzeichnet, daß**

   - die Flüssigkeit durch eine in sie eingetauchte UV-Tauchlampe (7) bestrahlt wird, bis der in der Probemenge enthaltene organische Kohlenstoff vollständig oxidiert ist, und

   - die zum Kohlenstoffgehalt proportionale Kohlendioxidkonzentration der Flüssigkeit im Zuge des Durchströmens der Meßzelle (3) dynamisch erfaßt wird, wobei

   - ein zur unbehandelten Flüssigkeit korrespondierender Grundwert (G) der Kohlendioxidkonzentration sowie ein sich im Anschluß an die Kohlenstoffoxidation im Zuge des Durchflusses der Meßzelle (3) durch die mit Kohlendioxid angereicherte Flüssigkeit einstellender Maximalwert (M) der Kohlendioxidkonzentration gemessen werden, und wobei

   - die vom Kohlenstoffgehalt in der Flüssigkeit abhängige Differenz (D) aus Maximalwert (M) und Grundwert (G) gebildet wird (D = M - G).

2. Verfahren zur Bestimmung des Kohlenstoff (TOC-) Gehaltes in Flüssigkeiten, insbesondere Reinstwasser, wonach eine Probemenge der zu untersuchenden Flüssigkeit in eine Reaktionskammer (1) eingeleitet und chargenweise durch UV-Bestrahlung zur Oxidation von Kohlenstoff zu im wesentlichen Kohlendioxid statisch behandelt wird, anschließend die Probemenge durch von außen in die Reaktionskammer (1) eintretende Flüssigkeit in eine an die Reaktionskammer (1) angeschlossene Meßzelle (3) überführt wird, in der ihre Leitfähigkeit gemessen wird, und dann aus Leitfähigkeitsmeßwerten der Kohlenstoff (TOC-) Gehalt ermittelt wird, **dadurch gekennzeichnet, daß**

   - die Flüssigkeit durch eine in sie eingetauchte UV-Tauchlampe (7) bestrahlt wird, bis der in der Probemenge enthaltene organische Kohlenstoff vollständig oxidiert ist, und

   - die zum Kohlenstoffgehalt proportionale Kohlendioxid konzentration der Flüssigkeit im Zuge des Durchströmens der Meßzelle (3) dynamisch erfaßt wird, wobei

   - ein zur unbehandelten Flüssigkeit korrespondierender Grundwert (G) der Kohlendioxidkonzentration gemessen wird und

   - das Integral über das um den Grundwert (G) verringerte Meßsignal als Meßfläche (F) ermittelt wird, wobei

   - die Fläche (F) ein Maß für den Kohlenstoffgehalt ist.

3. Verfahren nach Anspruch 1 oder 2, **dadurch gekennzeichnet, daß** als von außen in die Reaktionskammer (1) nach der Kohlenstoffoxidation eintretende Flüssigkeit die zu untersuchende Flüssigkeit eingesetzt wird.

4. Verfahren nach einem der Ansprüche 1 bis 3, **dadurch gekennzeichnet, daß** vor und/oder nach Ermittlung der Kohlendioxidkonzentration die Reaktionskammer (1) und die Meßzelle (3) mittels durchströmender Flüssigkeit gereinigt werden.

5. Vorrichtung zur Bestimmung des Kohlenstoff (TOC-)Gehaltes in Flüssigkeiten, vorzugsweise Reinstwasser, insbesondere zur Durchführung des Verfahrens nach einem der Ansprüche 1 bis 4, mit einer Reaktionskammer (1) mit Einlaß (2) zur Aufnahme einer Probemenge der zu untersuchenden Flüssigkeit und statischen Oxidation des hierin gelösten Kohlenstoffs, und mit einer an die Reaktionskammer (1) angeschlossenen Leitfähigkeits-Meßzelle (3), in die die Probemenge im Anschluß an die Kohlenstoffoxidation durch von außen in die Reaktionskammer (1) eintretende Flüssigkeit überführbar ist, **dadurch gekennzeichnet, daß**

   - in der Reaktionskammer (1) eine in die Flüssigkeit eingetauchte UV-Tauchlampe (7) angeordnet ist,

   - ausgangsseitig der Meßzelle (3) eine Absperreinrichtung vorgesehen ist, welche nach Einleitung der Probemenge in die Reaktionskammer (1) schließbar und nach statischer Oxidation des in der Probemenge gelösten Kohlenstoffs zu Kohlendioxid öffenbar ist, und

   - die zum Kohlenstoffgehalt proportionale Kohlendioxidkonzentration der Flüssigkeit im Zuge des Durchströmens der Meßzelle (3) dynamisch erfaßbar ist, wobei

   - mit Hilfe einer Steuer-/Auswerteeinrichtung (6) ein zur unbehandelten Flüssigkeit korrespondierender Grundwert (G) der Kohlendioxidkonzentration und nach der Kohlenstoffoxidation durch zeitliches Differenzieren der Maximalwert (M) sowie die zugehörige Differenz (D = M - G) oder durch Integrieren des um den Grundwert (G) verringerten Meßsignals die Fläche (F) ermittelbar sind.

6. Vorrichtung nach Anspruch 5, **dadurch gekennzeichnet, daß** die Absperreinrichtung (4) als elektromagnetisch steuerbares Ventil (4) ausgebildet ist.

7. Vorrichtung nach Anspruch 5 oder 6, **dadurch gekennzeichnet, daß** die Meßzelle (1) zur Bestimmung der Kohlendioxidkonzentration der Flüssigkeit einen oder mehrere Leitfähigkeitssensoren (5) aufweist.

8. Vorrichtung nach einem der Ansprüche 5 bis 7, **dadurch gekennzeichnet, daß** die Meßzelle (1) (rotations-)symmetrisch ausgebildet ist und einen Zufluß (8) sowie einen Abfluß (9) mit jeweils gleich großem Querschnitt aufweist.

9. Vorrichtung nach einem der Ansprüche 5 bis 8, **dadurch gekennzeichnet, daß** die Reaktionskammer (1) eine mittige, auf einer Symmetrieachse (S) angeordnete UV-Tauchlampe (7) aufweist, wobei die Reaktionskammer (1) und die UV-Tauchlampe (7) in Bezug auf die Symmetrieachse (S) rotationssymmetrisch ausgebildet sind.

10. Vorrichtung nach einem der Ansprüche 5 bis 9, **dadurch gekennzeichnet, daß** die Reaktionskammer (1) und die UV-Lampe (7) eine kompakte, zusammengehörige Baueinheit bilden.

11. Vorrichtung nach einem der Ansprüche 5 bis 10, **dadurch gekennzeichnet, daß** die Reaktionskammer (1) die Form eines rotierenden U-Rohrs mit vorzugsweise 1 bis 5 mm Querschnittslänge (1) aufweist.

12. Vorrichtung nach einem der Ansprüche 5 bis 11, **dadurch gekennzeichnet, daß** zur Steuerung der Durchflußrate durch die Meßzelle (3) zwischen Reaktionskammer (1) und Meßzelle (3) ein vorzugsweise einstellbares Reduzierventil (11) zwischengeschaltet ist.

13. Vorrichtung nach einem der Ansprüche 5 bis 12, **dadurch gekennzeichnet, daß** parallel zur Reaktionskammer (1) und Meßzelle (3) eine Bypassleitung (12) mit elektromagnetisch steuerbarem Ventil (13) vorgesehen ist.

14. Vorrichtung nach einem der Ansprüche 5 bis 13, **dadurch gekennzeichnet, daß** sämtliche Ventile (4, 13 sowie ggf. 11) zusammen mit dem oder den Leitfähigkeitssensoren (5) an eine Steuer-/Auswerteeinrichtung (6) angeschlossen sind.

**Claims**

1. Procedure for the determination of the total organic carbon (TOC) content of liquids, in particular that of ultra-pure water, whereby a sample of the liquid under investigation is directed into a reaction chamber (1) and is treated statically batch by batch by the use of UV radiation, for oxidising carbon mainly to carbon dioxide, the sample quantity is then transferred by liquid entering the reaction chamber (1) from the outside to a measuring cell (3) connected to the reaction chamber (1), where the conductivity is measured and then the carbon content (TOC) is found from the conductivity measurements, **characterised by** the fact that

   - the liquid is irradiated by a UV immersion lamp (7) submerged in it until the organic carbon contained in the sample quantity is completely oxidised, and

- the carbon dioxide concentration which is proportional to the carbon content of the liquid is dynamically registered as it flows through the measuring cell (3), wherein

- a base value (G) of the carbon dioxide concentration corresponding to the untreated liquid and a maximum value (M) of the enriched carbon dioxide concentration appearing after carbon oxidation in the course of the liquid enriched with carbon dioxide flowing through the measuring cell (3), are measured and wherein

- the difference (D), which is dependent upon the carbon content of the liquid, between the maximum value (M) and the base value (G) is found (D = M - G).

2. Procedure for the determination of the total organic carbon (TOC) content of liquids, in particular that of ultra-pure water, whereby a sample of the liquid under investigation is directed into a reaction chamber (1) and is treated statically batch by batch by the use of UV radiation, for oxidising carbon mainly to carbon dioxide, the sample quantity is then transferred by liquid entering the reaction chamber (1) from the outside to a measuring cell (3) connected to the reaction chamber (1), where the conductivity is measured and then the carbon content (TOC) is found from the conductivity measurements, **characterised by** the fact that

- the liquid is irradiated by a UV immersion lamp (7) submerged in it until the organic carbon contained in the sample quantity is completely oxidised, and

- the carbon dioxide concentration which is proportional to the carbon content of the liquid is dynamically registered as it flows through the measuring cell (3), wherein

- a base value (G) of the carbon dioxide concentration corresponding to the untreated liquid is measured and

- the integral of the signal reduced by the base value (G) is calculated as area (F), wherein

- the area (F) represents the carbon content.

3. Procedure in accordance with claim 1 or 2, **characterised by** the fact that the liquid under investigation is used as liquid entering the reaction chamber (1) from the outside after oxidation of the carbon.

4. Procedure in accordance with claims 1 to 3, **characterised by** the fact that before and/or after the determination of carbon dioxide concentration the reaction chamber (1) and the measuring cell (3) are cleaned by means of liquid flowing through them.

5. Apparatus for the determination of the total organic carbon (TOC) content of liquids, in particular that of ultra-pure water, especially for the execution of the procedure in accordance with claims 1 to 4, having a reaction chamber (1) with inlet (2) for accommodation of a sample quantity of the liquid under investigation and static oxidation of the carbon dissolved therein, and, connected to the reaction chamber (1), a measuring cell (3) into which the sample quantity can be transferred after oxidation of the carbon by liquid entering the reaction chamber (1) from the outside **characterised by** the fact that

- a UV immersion lamp (7) is mounted in the reaction chamber (1) and is submerged in the liquid,

- a cut-off device is disposed on the outlet side of the measuring cell (3), which can be closed after introduction of the sample quantity into the reaction chamber (1) and after static oxidation of the carbon dissolved in the sample quantity to carbon dioxide can be opened, and

- the carbon dioxide concentration which is proportional to the carbon content of the liquid can be dynamically registered as it flows through the measuring cell (3), wherein

- with the aid of a control/analysis unit (6), a base value (G) of the carbon dioxide concentration corresponding to the untreated liquid, and after carbon oxidation by differentiation over time the maximum value (M) as well as the related difference (D = M - G) or by integration of the signal reduced by the base value (G) the area (F) can be found.

6. Apparatus in accordance with claim 5, **characterised by** the fact that the cut-off device (4) has the form of an electro-magnetically controlled valve (4).

7. Apparatus in accordance with claim 5 or 6, **characterised by** the fact that the measuring cell (1) for determination of the carbon dioxide concentration of the liquid, exhibits one or more conductivity sensors (5).

8. Apparatus in accordance with claims 5 to 7, **characterised by** the fact that the measuring cell (1) is formed (rotationally) symmetrically and exhibits an inlet (8) and an outlet (9) which each have a cross-section of the same size.

9. Apparatus in accordance with claims 5 to 8, **characterised by** the fact that the reaction chamber (1) exhibits a UV immersion lamp (7) mounted in the middle on an axis of symmetry S, wherein the reaction chamber (1) and the UV immersion lamp (7) are rotationally symmetrically formed with regard to the axis of symmetry (S).

10. Apparatus in accordance with claims 5 to 9, **characterised by** the fact that the reaction chamber (1) and the UV immersion lamp (7) form a compact assembly unit, belonging together.

11. Apparatus in accordance with claims 5 to 10, **characterised by** the fact that the reaction chamber (1) has the form of a rotating U-pipe, with a cross-sectional dimension (1) preferably of 1 to 5 mm.

12. Apparatus in accordance with claims 5 to 11, **characterised by** the fact that a preferably adjustable choke valve (11) is fitted between the reaction chamber (1) and measuring cell (3) to control the rate of flow through the measuring cell (3).

13. Apparatus in accordance with claims 5 to 12, **characterised by** the fact that a bypass (12) with an electro-magnetically controlled valve (13) is fitted parallel to the reaction chamber (1) and measuring cell (3).

14. Apparatus in accordance with claims 5 to 13, **characterised by** the fact that all of the valves (4, 13 and possibly 11), together with the conductivity sensor or sensors (5), are connected to a control/analysis unit (6).

## Revendications

1. Procédé pour déterminer la teneur en carbone organique total (COT) dans des liquides, notamment de l'eau ultra-pure, selon lequel une quantité échantillon du liquide à examiner est versée dans une chambre de réaction (1) et traitée statiquement par charges par rayonnement UV pour oxyder le carbone en pour l'essentiel dioxyde de carbone, la quantité échantillon est ensuite transférée par du liquide entrant de l'extérieur dans la chambre de réaction (1) dans une cellule de mesure (3) reliée à la chambre de réaction (1), dans laquelle sa conductibilité est mesurée, puis la teneur en carbone organique total (COT) est déterminée à partir des valeurs de mesure de la conductibilité,
   **caractérisé en ce que**

   - le liquide est exposé au rayonnement d'une lampe plongeuse UV (7) immergée dans celui-ci jusqu'à ce que le carbone organique contenu dans la quantité échantillon soit complètement oxydé,
   - la concentration de dioxyde de carbone du liquide proportionnelle à la teneur en carbone est détectée dynamiquement pendant l'écoulement à travers la cellule de mesure (3),
   - on mesure une valeur de base (G) de la concentration de dioxyde de carbone correspondant au liquide non traité ainsi qu'une valeur maximale (M) de la concentration de dioxyde de carbone obtenue pendant l'écoulement à travers la cellule de mesure (3) par le liquide enrichi de dioxyde de carbone après l'oxydation du carbone, et
   - on forme la différence (D), dépendant de la teneur en carbone dans le liquide, entre la valeur maximale (M) et la valeur (G), (D = M - G).

2. Procédé pour déterminer la teneur en carbone organique total (COT) dans des liquides, notamment l'eau ultra-pure, selon lequel une quantité échantillon du liquide à examiner est versée dans une chambre de réaction (1) et traitée statiquement par charges par rayonnement UV pour oxyder le carbone en pour l'essentiel dioxyde de carbone, la quantité échantillon est ensuite transférée par du liquide entrant de l'extérieur dans la chambre de réaction (1) dans une cellule de mesure (3) reliée à la chambre de réaction (1), dans laquelle sa conductibilité est mesurée, puis la teneur en carbone organique total (COT) est déterminée à partir des valeurs de mesure de la conductibilité,
   **caractérisé en ce que**

   - le liquide est exposé au rayonnement d'une lampe plongeuse UV (7) immergée dans celui-ci jusqu'à ce que le carbone organique contenu dans la quantité échantillon soit complètement oxydé,
   - la concentration de dioxyde de carbone du liquide proportionnelle à la teneur en carbone est détectée dynamiquement pendant l'écoulement à travers la cellule de mesure (3),
   - une valeur de base (G) de la concentration de dioxyde de carbone correspondant au liquide non traité est mesurée, et
   - l'intégrale est déterminée comme surface de mesure (F) par le signal de mesure diminué de la valeur de base (G),
   - la surface (F) étant une mesure pour la teneur en carbone.

3. Procédé selon la revendication 1 ou 2,
   **caractérisé en ce que**
   comme liquide entrant de l'extérieur dans la chambre de réaction (1) après l'oxydation du carbone, on utilise le liquide à examiner.

**4.** Procédé selon l'une quelconque des revendications 1 à 3,
**caractérisé en ce qu'**
avant et/ou après la détermination de la concentration de dioxyde de carbone, la chambre de réaction (1) et la cellule de mesure (3) sont nettoyées au moyen d'un liquide les traversant.

**5.** Dispositif pour déterminer la teneur en carbone organique total (COT) dans des liquides, notamment de l'eau ultra-pure, en particulier pour la mise en oeuvre du procédé selon l'une quelconque des revendications 1 à 4, comprenant une chambre de réaction (1) avec une entrée (2) pour recevoir une quantité échantillon du liquide à examiner et pour l'oxydation statique du carbone dissout dans celui-ci, et comprenant une cellule de mesure de conductibilité (3) reliée à la chambre de réaction (1), dans laquelle la quantité échantillon peut être transférée par du liquide entrant de l'extérieur dans la chambre de réaction (1) après l'oxydation du carbone,
**caractérisé en ce que**

- une lampe plongeuse UV (7) immergée dans le liquide est disposée dans la chambre de réaction (1),
- côté sortie de la cellule de mesure (3) est prévu un dispositif de verrouillage que l'on peut fermer après l'introduction de la quantité échantillon dans la chambre de réaction (1) et ouvrir après l'oxydation statique du carbone dissout dans la quantité échantillon en dioxyde de carbone, et
- la concentration de dioxyde de carbone du liquide proportionnelle à la teneur en carbone peut être détectée dynamiquement pendant l'écoulement à travers la cellule de mesure (3),
- avec un dispositif de commande/d'exploitation (6) une valeur de base (G) de la concentration de dioxyde de carbone correspondant au liquide non traité et, après l'oxydation du carbone, par différenciation temporelle la valeur maximale (M) ainsi que la différence associée (D = M - G) ou par intégration du signal de mesure diminué de la valeur de base (G) la surface (F) sont déterminées.

**6.** Dispositif selon la figure 5,
**caractérisé en ce que**
le dispositif de verrouillage (4) présente la forme d'une vanne (4) à commande électromagnétique.

**7.** Dispositif selon la revendication 5 ou 6,
**caractérisé en ce que**
la cellule de mesure (1) pour déterminer la concentration de dioxyde de carbone du liquide présente un ou plusieurs capteurs de conductibilité (5).

**8.** Dispositif selon l'une quelconque des revendications 5 à 7,
**caractérisé en ce que**
la cellule de mesure (1) est configurée à symétrie (de rotation) et présente une entrée (8) ainsi qu'une sortie (9) dont les dimensions des sections transversales sont identiques.

**9.** Dispositif selon l'une quelconque des revendications 5 à 8,
**caractérisé en ce que**
la chambre de réaction (3) présente une lampe plongeuse UV (7) centrale disposée sur un axe de symétrie (S), la chambre de réaction (1) et la lampe plongeuse UV (7) étant configurées à symétrie de rotation par rapport à l'axe de symétrie (S).

**10.** Dispositif selon l'une quelconque des revendications 5 à 9,
**caractérisé en ce que**
la chambre de réaction (1) et la lampe UV (7) constituent un seul ensemble compact.

**11.** Dispositif selon l'une quelconque des revendications 5 à 10,
**caractérisé en ce que**
la chambre de réaction (1) présente la forme d'un tube rotatif en U avec une longueur de section transversale (1) de préférence comprise entre 1 et 5 mm.

**12.** Dispositif selon l'une quelconque des revendications 5 à 11,
**caractérisé en ce que**
pour commander le taux de débit à travers la cellule de mesure (3), une vanne réductrice (11) de préférence réglable est insérée entre la chambre de réaction (1) et la cellule de mesure (3).

**13.** Dispositif selon l'une quelconque des revendications 5 à 12,
**caractérisé en ce que**
parallèlement à la chambre de réaction (1) et la cellule de mesure (3) on prévoit une conduite by-pass (12) avec vanne (13) à commande électromagnétique.

**14.** Dispositif selon l'une quelconque des revendications 5 à 13,
**caractérisé en ce que**
toutes les vannes (4, 13 et le cas échéant 11) sont raccordées conjointement avec le ou les capteur(s) de conductibilité (5) à un dispositif de commande / d'exploitation (6).

*Fig.1*

Fig.2

D = M - G

*Fig. 3*